(19) Europäisches Patentamt
European Patent Office
Office européen des brevets

(11) Veröffentlichungsnummer: **0 318 902**
**A2**

# (12) EUROPÄISCHE PATENTANMELDUNG

(21) Anmeldenummer: **88119839.4**

(22) Anmeldetag: **29.11.88**

(51) Int. Cl.[4]: **A61K 31/40 , A61K 31/415 , A61K 31/42 , A61K 31/425 , A61K 31/44 , A61K 31/50**

(30) Priorität: **03.12.87 DE 3740985**

(43) Veröffentlichungstag der Anmeldung:
**07.06.89 Patentblatt 89/23**

(84) Benannte Vertragsstaaten:
**AT BE CH DE ES FR GB GR IT LI LU NL SE**

(71) Anmelder: **BOEHRINGER MANNHEIM GMBH**
**Sandhofer Strasse 116**
**D-6800 Mannheim 31(DE)**

(72) Erfinder: **Von der Saal, Wolfgang, Dr. rer. nat.**
**Neuer Burgweg 3**
**D-6900 Weinheim(DE)**
Erfinder: **Mertens, Alfred, Dr. rer. nat.**
**Beethovenstrasse 20**
**D-6905 Schriesheim(DE)**
Erfinder: **Hoelck, Jens-Peter, Dr. rer. nat.**
**Ph. Brunnemer Weg 33**
**D-6800 Mannheim 31(DE)**
Erfinder: **Boehm, Erwin, Dr. med.**
**Hinterer Rindweg 37**
**D-6802 Ladenburg(DE)**
Erfinder: **Martin, Ulrich, Dr. med.**
**D 3, 4**
**D-6800 Mannheim 1(DE)**

## (54) Verwendung linear anellierter Tricyclen als Hemmer der Erythrozytenaggregation.

(57) Verwendung von linear anellierten Tricyclen der allgemeinen Formel I

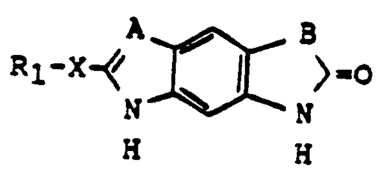

(I)

in der $R_1$ einen gegebenenfalls substituierten Phenylring oder einen gegebenenfalls substituierten heterocyclischen Fünfring mit 1 - 4 Heteroatomen oder einen heterocyclischen Sechsring mit 1 - 5 Heteroatomen darstellt, wobei die Heteroatome gleich oder verschieden sein können und Sauerstoff, Schwefel oder Stickstoff bedeuten und gewünschtenfalls an einem oder mehreren Stickstoffatomen ein Sauerstoffatomen tragen können,
oder für den Fall, daß X eine Bindung darstellt, $R_1$ neben den oben genannten Gruppen auch ein Wasserstoffatom, eine Alkyl-, Cycloalkyl-, Alkenyl-, Cycloalkenyl-, Alkinyl-, Halogenalkyl-, Alkoxyalkyl-, Carboxyalkyl-, Alkoxy-, carbonylalkyl-, eine Hydroxy-, Mercapto-, Amino-, Alkylmercapto-,

EP 0 318 902 A2

Formylaminoalkyl-, oder eine Pyridylcarbonylaminogruppe bedeutet,
X eine Bindung, eine Alkylen-, oder die Vinylengruppe bedeutet,
A ein Stickstoffatom oder die Gruppe $CR_5$ sein kann, wobei $R_5$ ein Wasserstoffatom, eine Alkyl-, Alkenyl-, Cycloalkyl-, Cycloalkenyl-, Cyan-, Alkylcarbonyl-, Alkoxycarbonyl-, Carboxy-, Aminocarbonyl-, Alkylaminocarbonyl-, Dialkylaminocarbonyl- oder Arylgruppe bedeutet,
B ein Sauerstoffatom, ein Schwefelatom oder die Gruppe $NR_6$ bedeutet, wobei $R_6$ ein Wasserstoffatom oder eine Alkylgruppe bedeutet, oder B die Gruppe $CR_7R_8$ sein kann, wobei $R_7$ ein Wasserstofatom, eine Alkyl-, Alkenyl-, oder eine Cycloalkylgruppe bedeutet, und $R_8$ ein Wasserstoffatom, eine Alkyl-, Alkenyl- oder Cyangruppe, eine durch eine Hydroxy-, Alkyl-, Alkoxy-, Amino-, Alkylamino-, Dialkylamino- oder Hydrazinogruppe substituierte Carbonylgruppe, oder $R_7$ und $R_8$ zusammen eine Alkyliden-oder Cycloalkylidengruppe bilden, oder $R_7$ und $R_8$ zusammen mit dem C-Atom, an das sie gebunden sind einen Spirocyclus bilden,
sowie deren pharmakologisch verträglichen Salze zur Behandlung von Krankheiten, bei denen in der Pathogenese die Erythrozytenaggregation eine wichtige Rolle spielt.

## Verwendung linear anellierter Tricyclen als Hemmer der Erythrozytenaggregation

Gegenstand der vorliegenden Erfindung ist die Verwendung von Verbindungen der allgemeinen Formel I,

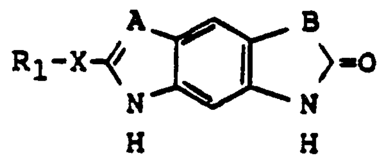

(I)

in welcher

$R_1$ einen Phenylring der allgemeinen Formel II darstellt,

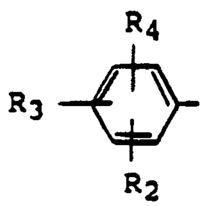

(II),

wobei $R_2$, $R_3$, $R_4$ gleich oder verschieden sein können und jeweils Wasserstoff, eine Alkansulfonyloxy-, Trifluormethansulfonyloxy-, Alkansulfonylamino-, Trifluormethansulfonylamino-, N-Alkyl-alkansulfonylamino-, N-Alkyltrifluormethansulfonylamino-, Alkylsulfenylmethyl-, Alkylsulfinylmethyl- oder Alkylsulfonylmethylgruppe, eine durch eine Hydroxy-, Alkoxy-, Amino-, Alkylamino- oder Dialkylaminogruppe substituierte Carbonylgruppe, eine durch eine Amino-, Alkylamino-, Dialkylamino- oder cyclische Iminogruppe substituierte Sulfonylgruppe, wobei eine Methylengruppe in 4-Stellung durch ein Schwefel- oder Sauerstoffatom ersetzt sein kann, eine Alkylcarbonylamino-, Aminocarbonylamino- oder Alkylaminocarbonylaminogruppe, eine Alkylmercapto-, Alkylsulfinyl- oder Alkylsulfonylgruppe, eine Nitro-, Halogen-, Amino-, Hydroxy-, Alkyl-, Alkoxy-, Alkenyloxy-, Alkinyloxy-, Cyanoalkyloxy-, Carboxyalkoxy-, Alkoxycarbonylalkyloxy-, Dialkylamino-, 1-Imidazolyl-, Trifluormethyl- oder Cyangruppe sein können,

oder einen heterocyclischen Fünfring mit 1-4 Heteroatomen oder einen heterocyclischen Sechsring mit 1-5 Heteroatomen darstellt, wobei die Heteroatome gleich oder verschieden sein können und Sauerstoff, Schwefel oder Stickstoff bedeuten und gewünschtenfalls an einem oder mehreren Stickstoffatomen ein Sauerstoffatom tragen können, und die Fünf- oder Sechsringe gegebenenfalls durch eine oder mehrere Alkyl-, Alkoxy, Alkylmercapto-, Hydroxy-, Nitro-, Amino-, Halogen- oder Cyangruppen substituiert sein können,

oder für den Fall, daß X eine Bindung darstellt, $R_1$ neben den oben genannten Gruppen auch ein Wasserstoffatom, eine Alkyl-, Cycloalkyl-, Alkenyl-, Cycloalkenyl-, Alkinyl-, Halogenalkyl-, Alkoxyalkyl-, Carboxylalkyl-, Alkoxy-carbonylalkyl-, eine Hydroxy-, Mercapto-, Amino-, Alkylmercapto-, Formylaminoalkyl-, oder eine Pyridylcarbonyl-aminogruppe bedeutet,

X eine Bindung, eine Alkylen-, oder die Vinylengruppe bedeutet,

A ein Stickstoffatom oder die Gruppe $\geq CR_5$ sein kann, wobei $R_5$ ein Wasserstoffatomen, eine Alkyl-, Alkenyl-, Cycloalkyl-, Cycloalkenyl, Cyan-, Alkylcarbonyl-, Alkoxycarbonyl-, Carboxy-, Aminocarbonyl-, Alkylaminocarbonyl-, Dialkylaminocarbonyl- oder Arylgruppe bedeutet,

B ein Sauerstoffatom, ein Schwefelatom oder die Gruppe $>NR_6$ bedeutet, wobei $R_6$ ein Wasserstoffatom oder eine Alkylgruppe bedeutet, oder B die Gruppe $>CR_7R_8$ sein kann, wobei $R_7$ ein Wasserstofatom, eine Alkyl-, Alkenyl-, oder eine Cycloalkylgruppe bedeutet, und $R_8$ ein Wasserstoffatom, eine Alkyl-, Alkenyl- oder Cyangruppe, eine durch eine Hydroxy-, Alkyl-, Alkoxy-, Amino-, Alkylamino-, Dialkylamino- oder Hydrazinogruppe substituierte Carbonylgruppe, oder $R_7$ und $R_8$ zusammen eine Alkyliden-oder Cycloalkylidengruppe bilden, oder $R_7$ und $R_8$ zusammen mit dem C-Atom, an das sie gebunden sind einen Spirocyclus bilden,

oder deren Tautomeren, optisch aktiven Formen und racemischen Gemischen, oder deren physiologisch

verträglichen Salzen anorganischer und organischer Säuren, zur Herstellung von Arzneimitteln mit erythrozytenaggregationshemmender Wirkung.

Die Verbindungen der allgemeinen Formel I, Verfahren zu ihrer Herstellung und deren Verwendung als Arzneimittel sind aus EP-A-0,161,632, EP-A-0,186,010, EP-A-0,189,103, EP-A-0,207,483, EP-A-0,214,592 und EP-A-0,216,165 bekannt. Die Verbindungen werden in diesen Anmeldungen mit den folgenden pharmakologischen Wirkungen beschrieben: Steigerung der Herzkraft, Blutdrucksenkung, Beeinflussung der Thrombozytenaggregation und Verbesserung der Mikrozirkulation.

Die in den oben genannten Anmeldungen beschriebenen Verbindungen eignen sich jedoch primär für die Behandlung solcher Krankheiten, bei denen eine Steigerung der Herzkraft und eine Senkung des Blutdrucks im Vordergrund stehen, wobei als zusätzlicher pharmakologischer Effekt die Verbesserung des Flußes in der Mikrozirkulation durch die Thrombozytenaggregationshemmung einerseits und durch die Zunahme der Kontraktilität des Herzens andererseits erreicht wird.

Überraschenderweise wurde nun gefunden, daß Verbindungen der allgemeinen Formel I eine ausgeprägte Wirkung hinsichtlich der Hemmung der Erythrozytenaggregation aufweisen. Damit eignen sich diese Verbindungen auch zur Behandlung von solchen Krankheiten, bei denen in der Pathogenese die Erythrozytenaggregation eine wichtige Rolle spielt, wie z.B. periphere, coronare oder zerebrale Durchblutungsstörungen, Schockzustände, degenerative Gefäßerkrankungen, rheumatische Erkrankungen, verschiedene Arten von Ulcera, nekrotischen Prozessen in Tumoren, degenerative Störungen der Retina, Nerven und Muskeln, oder von verschiedenen Hautkrankheiten. Insbesondere kommt auch die Behandlung von arteriellen Verschlußkrankheiten, ischämischen Zuständen, venöser Insuffizienz oder Diabetes mellitus in Frage. Mit Hilfe dieser Verbindungen können nun auch solche Erkrankungen behandelt werden, die nicht mit einer Schwächung des Herzens oder einer Blutdruckerhöhung verbunden sind, sondern für deren Ursache eine gesteigerte Erythrozytenaggregationsneigung eine bedeutende Rolle spielt. Damit wird ein neues erfolgversprechendes therapeutisches Prinzip realisiert, da diese Verbindungen die ersten Verbindungen darstellen, die in pharmakologisch relevanten Konzentrationen die Erythrozytenaggregation vermindern, und rheologisch wirksam sind.

Bedeutet $R_1$ eine Phenylring der allgemeinen Formel II, so kann der Alkylteil der bei $R_2$, $R_3$ und $R_4$ genannten Substituenten 1-5 Kohlenstoffatome enthalten, vorzugsweise 1-4 Kohlenstoffatome. Bevorzugt in diesem Sinne sind beispielsweise die Methansulfonyloxy-, Ethansulfonyloxy-, n-Propansulfonyloxy-, Isopropansulfonyloxy-, Trifluormethansulfonyloxy-, Methylsulfenylmethyl-, Ethylsulfenylmethyl-, n-Propylsulfenylmethyl-, Methylsulfinylmethyl-, Ethylsulfinylmethyl-, n-Propylsulfinylmethyl-, Methylsulfonylmethyl-, Ethylsulfonylmethyl-, n-Propylsulfonylmethyl-, Methansulfonylamino-, Ethansulfonylamino-, n-Propansulfonylamino-, Trifluormethansulfonylamino-, n-Methylmethansulfonylamino-, N-Ethyl-methansulfonylamino-, N-Methyl-ethansulfonylamino-, N-Ethyl-ethansulfonylamino-, N-Isopropylethansulfonylamino-, N-Methyl-n-propansulfonylamino-, N-n-Propyl-n-propansulfonylamino-, N-Methyltrifluormethansulfonylamino-, N-Ethyl-trifluormethan sulfonylamino-, N-Isopropyl-trifluormethansulfonylamino-, Methoxycarbonyl-, Ethoxycarbonyl-, Propoxycarbonyl-, Isopropoxycarbonyl-, Methylaminocarbonyl-, Ethylaminocarbonyl-, Dimethylaminocarbonyl, Di-n-propylaminocarbonyl-, N-Methyl-ethylaminocarbonyl-, Trifluormethyl-, Methylaminosulfonyl-, Ethylaminosulfonyl-, n-Propylaminosulfonyl-, n-Butylaminosulfonyl-, n-Pentylaminosulfonyl-, Dimethylaminosulfonyl-, Diethylaminosulfonyl-, Di-n-propylaminosulfonyl-, N-Methylisopropylaminosulfonyl-, Acetylamino-, Propionylamino-, Methylcarbonylamino-, Ethylaminocarbonylamino- oder Propylaminocarbonylaminogruppe, eine Methyl-, Ethyl-, Propyl-, Methoxy-, Ethoxy-, Propyloxy-, Allyloxy-, 2-Butenyloxy-, 3-Butenyloxy-, 2-Pentenyloxy-, Propargyloxy-, 2-Butinyloxy-, 3-Butinyloxy-, Cyanmethyloxy-, Cyanethyloxy-, Methoxycarbonylmethyloxy-, Methoxycarbonylethyloxy-, Methylmercapto-, Ethylmercapto-, Methylsulfinyl-, Ethylsulfinyl-, Methylsulfonyl oder Ethylsulfonylgruppe.

Bei Sulfonylgruppen, die durch cyclische Iminogruppen substituiert sein können, sind bevorzugt die Morpholino-, Thiomorpholino-, Pyrrolidino-, Piperidino- und Hexamethyleniminosulfonylgruppen.

Insbesondere sind bevorzugt für

$R_2$ Wasserstoff, eine Alkylsulfonyloxy-, Trifluormethylsulfonyloxy-, Alkylsulfenylmethyl-, Alkylsulfinylmethyl-, Alkylsulfonylmethyl-, Alkylsulfonylamino-, N-Alkyl-alkylsulfonylamino-, Trifluormethylsulfonylamino- oder N-Alkyltrifluormethylsulfonylaminogruppe, eine durch eine Hydroxy-, Alkoxy-, Amino-, Alkylamino- oder Dialkylaminogruppe substituierte Carbonylgruppe oder eine durch eine Amino-, Dialkylamino- oder Morpholinogruppe substituierte Sulfonylgruppe, wobei jeder der vorstehend genannten Alkylteile 1 oder 2 Kohlenstoffatome enthalten kann, eine Nitro-, Cyan- oder Alkylaminosulfonylgruppe mit 1-4 Kohlenstoffatomen, eine Alkylcarbonylamino-, Aminocarbonylamino- oder N-Alkyl-aminocarbonylaminogruppe, eine Alkylmercapto-, Alkylsulfinyl- oder Alkylsulfonylgruppe, wobei jeder der vorgenannten Alkylteile 1 oder 2 Kohlenstoffatome enthalten kann, eine Halogen-, Amino-, Hydroxy-, Dialkylamino-, Alkyl-, Alkoxy-, Alkenyloxy- oder Alkinyloxygruppe vorzugsweise mit 1-3 Kohlenstoffatomen, eine Cyanmethyloxy- oder Methoxycarbonylmethyloxygruppe, die Trifluormethylgruppe oder die 1-Imidazolylgruppe, für $R_3$ Wasserstoff, eine Alkylgruppe mit 1-3 Kohlenstoffatomen, eine Alkoxy-oder Dialkylaminogruppe mit 1 oder 2 Kohlenstoffatomen in jedem Alkylteil oder ein Halogenatom und
für $R_4$ Wasserstoff oder die Methoxygruppe.

Der Phenylteil kann 1 bis 3 genannten Substituenten tragen.

Bevorzugte monosubstituierte Phenylverbindungen sind die Hydroxy-, $C_1$-$C_3$ Alkyl-, $C_1$-$C_3$ Alkoxy-, Allyloxy-, Propargyloxy-, Cyanmethyloxy-, Methoxycarbonylmethyloxy-, Halogen-, Nitro-, Cyan-, Aminocarbonyl-, Methoxycarbonyl-, Amino-, $C_1$-$C_3$ Dialkylamino-, $C_1$-$C_3$ Alkylmercapto, $C_1$-$C_3$ Alkylsulfinyl-, $C_1$-$C_3$ Alkylsulfonyl-, $C_1$-$C_3$ Alkylsulfonyloxy-, und die 1-Imidazolyl-phenyle, wobei der Substituent in 2-, 3- oder 4-Stellung stehen kann.

Bevorzugte disubstituierte Phenyle enthalten als Substituenten eine Alkansulfonyloxy-, Trifluormethylsulfonyloxy-, Alkylsulfenylmethyl-, Alkylsulfinylmethyl-, Alkylsulfonylmethyl-, Alkylsulfonylamino-, N-Alkyl-alkylsulfonylamino-, Trifluormethylsulfonylamino- oder N-Alkyl-trifluormethylsulfonylaminogruppe, eine durch eine Hydroxy-, Alkoxy-, Amino-, Alkylamino- oder Dialkylaminogruppe substituierte Carbonylgruppe oder eine durch eine Amino-, Dialkylamino-oder Morpholinogruppe substituierte Sulfonylgruppe, eine Alkylaminosulfonyl-, Alkylcarbonylamino-, Aminocarbonylamino- oder N-Alkyl-aminocarbonylaminogruppe, eine Hydroxy-, Alkyl-, Alkoxy-, Allyloxy-, Propargyloxy-, Cyanmethyloxy-, Methoxycarbonylmethyloxy-, Cyan-, Halogen-, Nitro-, Amino-, Dialkylamino-, Alkylmercapto-, Alkylsulfinyl-, Alkylsulfonyl- oder eine 1-Imidazolylgruppe, wobei die beiden Substituenten gleich oder verschieden sein können und in 2,3-, 2,4-, 2,5-, 2,6-, 3,4- und 3,5-Stellung, bevorzugt jedoch in 2,4-, 2,5- und 3,4-Stellung stehen können und die vorgenannten Alkylreste, allein oder in Kombination mit anderen Resten. 1-3 C-Atomen aufweisen können.

Bevorzugte trisubstituierte Phenyl enthalten Hydroxy- und Methoxygruppen als Substituenten.

Bedeutet $R_1$ einen heterocyclischen Fünfring mit 1-4 Heteroatomen oder einen heterocyclischen Sechsring mit 1-5 Heteroatomen, wobei die Heteroatome der vorgenannten Fünf-oder Sechsringe gleich oder veschieden sein können und Stickstoff, Sauerstoff oder Schwefel bedeuten und gegebenenfalls an einem oder mehreren Stickstoffatomen ein Sauerstoff tragen können, so sind in diesem Sinne bevorzugt der Pyrrol-, Furan-, Thiophen-, Pyrazol-, Imidazol-, Thiazol-, Isothiazol-, Oxazol-, Isoxazol-, Triazol-, Tetrazol-, Thiadiazol-, Oxadiazol-, Pyrazin-, N,N′-Dioxypyrazin-, Pyrimidin-, N,N′-Dioxy-pyrimidin-, Pyridazin-, Oxazin-, Thiazin-, Triazin-, Tetrazin-, Pyridin-, und N-Oxy-pyridinrest.

Alkyl-, Alkoxy- und Alkylmercapto-Substituenten in den heterocyclischen Fünf- und Sechsringen können 1-6, vorzugsweise 1-4 Kohlenstoffatome enthalten. Bevorzugt ist der Methyl-, Ethyl-, Methoxy-, Ethoxy-, Methylmercapto-und Ethylmercaptorest. Unter Halogen ist Fluor, Chlor und Brom, vorzugsweise Chlor zu verstehen.

Bedeutet X eine Bindung und $R_1$ einen Alkyl-, Alkenyl- oder Alkinylrest, so sind darunter gerade oder verzweigte Ketten mit bis zu acht C-Atomen zu verstehen. Bevorzugt in diesem Sinne ist der Methyl-, Ethyl-, Propyl-, Butyl-, Pentyl-, Hexyl-, Vinyl-, Propenyl- und Propinylrest. Bedeutet X eine Bindung und $R_1$ einen Cycloalkyl- oder Cycloalkenylrest, so sind darunter Ringe mit drei bis sieben Gliedern zu verstehen. Bevorzugt in diesem Sinne ist der Cyclopropyl-, Cyclobutyl-, Cyclopentyl-, Cyclohexyl-, Cyclopentenyl- und Cyclohexenylrest. Bedeutet X eine Bindung und $R_1$ einen Alkoxyalkyl-, Carboxyalkyl-, Alkoxycarbonylalkyl-, Hydroxyalkylrest, so können die Alkyl- oder Alkoxygruppen 1 bis 6 C-Atome enthalten.

Bevorzugt in diesem Sinne ist der Ethoxymethyl-, Methoxyethyl-, Ethoxyethyl-, Carboxymethyl-, Carboxypropyl-, Carboxybutyl-, Methoxycarbonylmethyl-, Methoxycarbonylethyl-, Methoxycarbonylpropyl-, Ethoxycarbonylmethyl-, Ethoxycarbonylethyl-, Ethoxycarbonylpropyl-, Propoxycarbonylethyl-, Hydroxymethyl-, Hydroxyethyl-, Hydroxypropyl-, Hydroxybutylrest.

Für X ist eine Bindung, eine Methylen-, Ethylen- oder die Vinylengruppe bevorzugt.

Bedeutet A die Gruppe $\geq CR_5$ und $R_5$ eine Alkylcarbonyl-, Alkoxycarbonyl-, Aminocarbonyl-, Alkylaminocarbonyl-, oder Dialkylaminocarbonylgruppe, so können die genannten Alkyl-oder Alkoxyreste 1-7 Kohlenstoffatome, vorzugsweise 1-5 Kohlenstoffatome enthalten. Bevorzugt sind dabei die Acetyl-, Propionyl-, Methoxycarbonyl-, Ethoxycarbonyl-, Aminocarbonyl-, Methylaminocarbonyl- oder Dimethylaminocarbonylgruppe.

Bedeutet B die Gruppe $>NR_6$ und $R_6$ eine Alkylgruppe, so sind bevorzugt die Methyl-, Ethyl-, Propyl-, Isopropyl-, Butyl-, 2-Butyl- und die 1,1-Dimethyl-ethylgruppe.

Bedeutet B die Gruppe $>CR_7R_8$ und $R_7$ und/oder $R_8$ eine Alkyl-, Cycloalkyl-, Alkenyl- oder eine durch eine Alkyl-, Alkoxy-, Alkylamino- oder Dialkylaminogruppe substituierte Carbonylgruppe, so kann jeder der vorgenannten Alkyl- oder Alkenylteile geradkettig oder verzweigt sein und 1-6 bzw. 2-6 Kohlenstoffatome und der genannte Cycloalkylteil 3-7 Kohlenstoffatome enthalten.
Bevorzugt in diesem Sinne ist für $R_7$ ein Wasserstoffatom, die Methyl-, Ethyl-, Isopropyl-, 3-Pentyl-,

Cyclopentyl-oder Cyclohexylgruppe. $R_8$ kann vorzugsweise das Wasserstoffatom, eine Methyl-, Ethyl-, Isopropyl-, 3-Pentyl-, Cyan-, Carboxy-, Acetyl-, Propinyl-, Methoxycarbonyl-, Ethoxycarbonyl-, Aminocarbonyl-, Methylaminocarbonyl-, Dimethylaminocarbonyl- und Hydrazinocarbonylgruppe darstellen.

Bilden $R_7$ und $R_8$ zusammen mit dem C-Atom, an das sie gebunden sind, ein Cycloalkylring, so handelt es sich dabei vorzugsweise um die Spirocyclopropyl-, Spirocyclobutyl-, Spirocyclopentyl- und Spirocyclohexylgruppe. Bilden $R_7$ und $R_8$ zusammen eine Alkyliden- oder Cycloalkylidengruppe, so ist dabei die Isopropyliden- oder Cyclohexylidengruppe bevorzugt.

Besonders bevorzugt sind Verbindungen der allgemeinen Formel I, in der $R_1$ den Phenylrest der allgemeinen Formel II bedeutet, in der

$R_2$ Wasserstoff, die Methansulfonyloxy-, Trifluormethansulfonyloxy-, Methansulfonylamino-, Trifluormethansulfonylamino-, Methansulfonyl-methylamino-, Trifluormethansulfonyl-methylamino-, Methylsuflinylmethyl-, Methylsulfonylmethyl-, Aminocarbonyl-, Aminosulfonyl-, Methylaminosulfonyl-, Dimethylaminosulfonyl-, Acetylamino-, Methylmercapto-, Methylsulfinyl-, Methylsulfonyl-, Hydroxy-, Allyloxy-, Methyl-, Methoxy-, Propargyloxy-, Cyanmethyl-, oxy-, Methoxycarbonyl-methyloxy-, Cyan-, Chlor-, Nitro-, Amino-, Dimethylamino-, Trifluormethyl- oder die 1-Imidazolylgruppe,

$R_3$ Wasserstoff, die Methyl-, Methoxy-, die Hydroxy-, Dimethylamino- oder Chlorgruppe bedeutet,

$R_4$ Wasserstoff oder die Methoxygruppe ist

oder

$R_1$ den Pyrrol-, Furan-, Thiphen-, Pyrazol-, Imidazol-, Isothiazol-, Thiazol-, Oxazol-, Triazol-, Tetrazol-, Thiadiazol-, Isoxazol-, Oxadiazol-, Pyridin-, N-Oxy-pyridin-, Pyrazin-, Pyrimidin-, N,N′-Dioxypyrimidin-, Pyridazin-, Oxazin-, Thiazin-, Triazin- oder Tetrazinrest darstellt, sowie deren Methyl-, Ethyl-, Methoxy-, Ethoxy-, Methylmercapto-, Ethylmercapto- und chlorsubstituierten Derivate,

oder

$R_1$ für den Fall, daß X eine Bindung darstellt, neben den genannten Gruppen auch ein Wasserstoffatom, die Methyl-, Ethyl-, Propyl-, Butyl-, Pentyl-, Hexyl-, Propenyl-, Cyclopentenyl-, Cyclohexyl-, Trifluormethyl-, Hydroxy-, Mercapto-, Methylmercapto-, Aminogruppe bedeutet,

X eine Bindung, die Ethylen- oder Vinylengruppe bedeutet,

A ein Stickstoffatom oder die -Ċ H-Gruppe bedeutet,

B ein Sauerstoffatom, Schwefelatom, oder die Gruppe $>NR_6$ bedeutet, wobei $R_6$ die Methyl-, Ethyl- oder Propylgruppe bedeutet, oder B die Gruppe $CR_7R_8$ bedeutet, wobei $R_7$ ein Wasserstoffatom, oder die Methylgruppe darstellt und $R_8$ die Methyl-, Ethyl- oder Isopropylgruppe bedeutet, oder $R_7$ und $R_8$ zusammen mit dem C-Atom, an das sie gebunden sind, einen Spirocyclopentylring darstellen.

Zur Herstellung von Arzneimitteln werden die Substanzen der allgemeinen Formel I in an sich bekannter Weise mit geeigneten pharmazeutischen Trägersubstanzen, Aroma-, Geschmacks- und Farbstoffen gemischt und beispielsweise als Tabletten oder Dragees ausgeformt oder unter Zugabe entsprechender Hilfsstoffe in Wasser oder Öl, wie z.B. Olivenöl, suspendiert oder gelöst.

Die Substanzen der allgemeinen Formel I und ihre Salze können in flüssiger oder fester Form enteral oder parenteral appliziert werden. Als Injektionsmedium kommt vorzugsweise Wasser zur Anwendung, welches die bei Injektionslösungen üblichen Zusätze wie Stabilisierungsmittel, Lösungsvermittler oder Puffer enthält.

Derartige Zusätze sind z.B. Tartrat- und Citratpuffer, Ethanol, Komplexbildner (wie Ethylendiamintetraessigsäure und deren nicht toxische Salze) und hochmolekulare Polymere (wie flüssiges Polyethylenoxid) zur Viskositätsregulierung. Feste Trägerstoffe sind z.B. Stärke, Lactose, Mannit, Methylcellulose, Talkum, hochdisperse Kieselsäuren, hochmolekulare Fettsäuren (wie Stearinsäure), Gelatine, Agar-Agar, Calciumphosphat, Magnesiumstearat, tierische und pflanzliche Fette und feste hochmolekulare Polymere (wie Polyethylenglykole). Für orale Applikation geeignete Zubereitungen können gewünschtenfalls Geschmacks- und Süßstoffe enthalten.

Die Verbindungen werden üblicherweise in Mengen von 10-2000 mg pro Tag bezogen auf 75 kg Körpergewicht appliziert. Bevorzugt ist es, 2-4 mal pro Tag 1-2 Tabletten mit einem Wirkstoffgehalt von 5-500 mg zu verabreichen. Die Tabletten können auch retardiert sein, wodurch nur noch 1 mal pro Tag 1-2 Tabletten mit 20-1000 mg Wirkstoff gegeben werden müssen. Der Wirkstoff kann auch durch Injektion 1-8 mal pro Tag bzw. durch Dauerinfusion gegeben werden, wobei Mengen von 10-1000 mg pro Tag normalerweise ausreichen.

Die Bestimmung der Erythrozytenaggregation erfolgte mit dem Mini-Erythrozyten-Aggregometer der Fa. Myrenne, Rötgen (Kiesewetter et al., Biomed. Technik 27, 209-213 (1982)). Als Maß gibt dieses Gerät einen dimensionslosen Index an, der mit steigender Aggregationstendenz zunimmt.

Die Untersuchungen wurden mit Humanblut gesunder Spender durchgeführt. Das Blut wurde auf einen Hämatokrit von 45 % eingestellt mit der Kontrollösung oder einer Substanzlösung versetzt und inkubiert.

Dann wurde die Erythrozyten-Aggregation gemessen. Jede Substanz wurde in einer Konzentration von $10^{-5}$ molar untersucht. Pro Substanz wurden zwei Versuche mit dem Blut zweier Spender durchgeführt. Es wurde die Differenz der Aggregationsindizes zwischen dem Ausgangswert der Kontrollösung und den Werten mit der Substanzlösung errechnet (ΔE).

In der folgenden Tabelle sind die vorliegenden Befunde zur Erythrozyten-Aggregation (ΔE) aufgelistet. Je niederiger der aufgeführte Wert ist, desto wirksamer ist die Substanz. Venoruton , ein Gemisch aus verschiedenen 0-(beta-Hydroxyethyl)rutosiden, bewirkt dagegen bei einer vergleichbaren Konzentration von $1.7 . 10^{-5}$ M lediglich eine Änderung des Erythrozyten-Aggregationsindexes um -0.4. Selbst bei einer Konzentration von $1.7 . 10^{-3}$ M beträgt die Änderung nur -3.9 ± 0.9.

Venoruton soll die Tendenz der Erythrozyten-Aggregation hemmen. (Schmid-Schönbein et al., VASA $\underline{4}$ 263-270 (1975)).

Im Vergleich zum Stand der Technik hemmen die genannten Substanzen der vorliegenden Erfindung die Erythrozyten-Aggregation deutlich stärker.

| Nr. | Verbindung | Schmp. [°C] | ΔE [$10^{-5}$M] |
|---|---|---|---|
| 1 | 7,7-Dimethyl-2-(4-pyridyl)-6,7-dihydro-3H,5H-pyrrolo[2,3-f]benzimidazol-6-on . 4 $H_2O$ | 215 | -8 |
| 2 | 7,7-Dimethyl-2-(2-pyridyl)-6,7-dihydro-3H,5H-pyrrolo[2,3-f]benzimidazol-6-on . 0.3 $H_2O$ | 182-187 | -4 |
| 3 | 7,7-Dimethyl-2-(3-pyridyl)-6,7-dihydro-3H,5H-pyrrolo[2,3-f]benzimidazol-6-on . 3 $H_2O$ | 331-335 | -4 |
| 4 | 7,7-Dimethyl-2-(4-(2-methylpyridyl))-6,7-dihydro-3H,5H-pyrrolo-[2,3-f]benzimidazol-6-on . 0.6 $H_2O$ | 311-313 | -9 |
| 5 | 7,7-Dimethyl-2-(4-(2-hydroxypyridyl))-6,7-dihydro-3H,5H-[2,3-f]benzimidazol-6-on . 2 $H_2O$ | >360 | -1 |
| 6 | 7,7-Dimethyl-2-(4-(2-chlor-pyridyl))-6,7-dihydro-3H,5H-pyrrolo-[2,3-f]benzimidazol-6-on | 341-344 | -11 |
| 7 | 7,7-Dimethyl-2-(2-(3-pyridyl)-ethenyl)-6,7-dihydro-3H,5H-pyrrolo[2,3-f]benzimidazol-6-on . 0.6 $H_2O$ | 203-207 | -8 |
| 8 | 7,7-Dimethyl-2-(2-(4-pyridyl)-ethyl)-6,7-dihydro-3H,5H-pyrrolo-[2,3-f]benzimidazol-6-on | 150-154 | -1 |
| 9 | 2'-(4-Pyridyl)-spiro[cyclopentan-1,7'-6,7'-dihydro-3'H,5'H-pyrrolo-[2',3'-f]benzimidazol-6'-on . 0.3 $H_2O$ | >365 | -9 |
| 10 | 7,7-Dimethyl-2-(3-(6-methyl-pyridyl))-6,7-dihydro-3H,5H-pyrrolo-[2,3-f]benzimidazol-6-on | >360 | -4 |

| Nr. | Verbindung | Schmp. [°C] | Δ E [$10^{-5}$M] |
|---|---|---|---|
| 11 | 7,7-Dimethyl-2-(3-(2-methoxy-6-methyl)pyridyl)-6,7-dihydro-3H,5H-pyrrolo[2,3-f]benzimidazol-6-on | 296-298 | -9 |
| 12 | 7,7-Dimethyl-2-(4-N-oxy-pyridyl)-6,7-dihydro-3H,5H-pyrrolo[2,3-f]-benzimidazol-6-on . 3 $H_2O$ | 260-262 | -2 |
| 13 | 7-Ethyl-2-(4-pyridyl)-6,7-dihydro-3H,5H-pyrrolo[2,3-f]benzimidazol-6-on | 270-272 | -2 |
| 14 | 7-Ethyl-2-(3-pyridyl)-6,7-dihydro-3H,5H-pyrrolo[2,3-f]benzimidazol-6-on | >300 | -2 |
| 15 | 7-(2-Propyl)-2-(4-pyridyl)-6,7-dihydro-3H,5H-pyrrolo[2,3-f]benzimidazol-6-on | 215-220 | -2 |
| 16 | 7-Cyclopentyl-2-(4-pyridyl)-6,7-dihydro-3H,5H-pyrrolo[2,3-f]benzimidazol-6-on | 200-204 | -2 |
| 17 | 7,7-Diethyl-2-(4-pyridyl)-6,7-dihydro-3H,5H-pyrrolo[2,3-f]benzimidazol-6-on | 216-219 | -5 |
| 18 | 7-Ethoxycarbonyl-7-methyl-2-(4-pyridyl)-6,7-dihydro-3H,5H-pyrrolo-[2,3-f]benzimidazol-6-on . HCl | 288-290 | -2 |
| 19 | 7-(2-Methyl-propyl)-2-(4-pyridyl)-6,7-dihydro-3H,5H-pyrrolo[2,3-f]-benzimidazol-6-on x 1.5 $H_2O$ | 200-202 | -1 |
| 20 | 7,7-Dimethyl-2-(4-(3-hydroxy-pyridyl)9-6,7-dihydro-3H-5H-pyrrolo[2,3-f]benzmimidazol-6-on | >300 | -8 |
| 21 | 7,7-Dimethyl-2-(2-(5-n-butyl-pyridyl))-6,7-dihydro-3H,5H-pyrrolo[2,3-f]benzimidazol-6-on | 176-178 | -7 |
| 22 | 7,7-Dimethyl-2-(2-furanyl)-6,7-dihydro-3H,5H-pyrrolo[2,3-f]benzimidazol-6-on | 311-316 | -5 |
| 23 | 7,7-Dimethyl-2-(2-thienyl)-6,7-dihydro-3H,5H-pyrrolo[2,3-f]benzimidazol-6-on | 332-336 | -8 |

| Nr. | Verbindung | Schmp. [°C] | ΔE [$10^{-5}$M] |
|---|---|---|---|
| 24 | 7,7-Dimethyl-2-(2-pyrazinyl)-6,7-dihydro-3H,5H-pyrrolo[2,3-f]benzimidazol-6-on | >300 | -2 |
| 25 | 7,7-Dimethyl-2-(4-thiazolyl)-6,7-dihydro-3H,5H-pyrrolo[2,3-f]benzimidazol-6-on | 280 | -3 |
| 26 | 7,7-Dimethyl-2-(2-methyl-pyrimidin-5-yl)-6,7-dihydro-3H,5H-pyrrolo-[2,3-f]benzimidazol-6-on | >350 | -4 |
| 27 | 7,7-Dimethyl-2-(1,2,4-1H-triazol-3-yl)-6,7-dihydroi-3H,5H-pyrrolo-[2,3-f]benzimidazol-6-on | >350 | -3 |
| 28 | 7,7-Dimethyl-2-(2-methyl-oxazol-4-yl)-6,7-dihydro-3H,5H-pyrrolo-[2,3-f]benzimidazol-6-on | 315-318 | -2 |
| 29 | 7-Methyl-7-ethoxycarbonyl-2-(2-pyrazinyl)-6,7-dihydro-3H,5H-pyrrolo[2,3-f]benzimidazol-6-on | 213-214 | -3 |
| 30 | 7,7-Dimethyl-2-(2-thienyl-methyl)-6,7-dihydro-3H,5H-pyrrolo[2,3-f]-benzimidazol-6-on | 233-235 | -3 |
| 31 | 7,7-Dimethyl-2-(1,2,3-thiadiazol-4-yl)-6,7-dihydro-3H,5H-pyrrolo-[2,3-f]benzimidazol-6-on | >300 | -3 |
| 32 | 7,7-Dimethyl-2-(1,2,3-thiadiazol-5-yl)-6,7-dihydro-3H,5H-pyrrolo-[2,3-f]benzimidazol-6-on | 286-290 | -9 |
| 33 | 7,7-Dimethyl-2-(2-methylmercapto-1,3,4-oxadiazol-5-yl)-6,7-dihydro-3H,5H-pyrrolo[2,3-f]benzimidazol-6-on | 311 | -3 |
| 34 | 7,7-Dimethyl-2-(4-methoxycarbonyl-1,2,3-1H-triazol-5-yl)-6,7-dihydro-3H,5H-pyrrolo[2,3-f]benzimidazol-6-on | >300 | -1 |

| Nr. | Verbindung | Schmp. [°C] | ΔE [$10^{-5}$M] |
|---|---|---|---|
| 35 | 2'-(4-Pyridazinyl)spiro[cyclopentan-1,7'-6',7'-dihydro-3'H,5'H-pyrrolo[2',3'-f]benzimidazol]-6'-on | 365-367 | -8 |
| 36 | 7-Ethyl-2-(4-pyridazinyl)-6,7-dihydro-3H,5H-pyrrolo-[2,3-f]benzimidazol-6-on | >300 | -5 |
| 37 | 2'-(2-Furanyl)spiro[cyclopentan-1,7'-6',7'-dihydro-3'H,5'H-pyrrolo-[2',3'-f]benzimidazol]-6'-on | >300 | -9 |
| 38 | 7,7-Dimethyl-2-(4-nitrophenyl)-6,7-dihydro-3H,5H-pyrrolo[2,3-f]benzimidazol-6-on | >300 | -5 |
| 39 | 7,7-Dimethyl-2-(4-aminophenyl)-6,7-dihydro-3H,5H-pyrrolo[2,3-f]-benzimidazol-6-on | 225-230 | -6 |
| 40 | 7,7-Dimethyl-2-(2-hydroxyphenyl)-6,7-dihydro-3H,5H-pyrrolo[2,3-f]-benzimidazol-6-on | 371-373 | -9 |
| 41 | 7,7-Dimethyl-2-(3,4-dichlorphenyl)-6,7-dihydro-3H,5H-pyrrolo[2,3-f]-benzimidazol-6-on | >300 | -10 |
| 42 | 7,7-Dimethyl-2-(2-phenyl-vinyl)-6,7-dihydro-3H,5H-pyrrolo[2,3-f]-benzimidazol-6-on | 320-325 | -10 |
| 43 | 7,7-Dimethyl-2-(2-methoxy-4-methylsulfinylphenyl)-6,7-dihydro-3H,5H-pyrrolo[2,3-f]benzimidazol-6-on | 217-220 | -10 |
| 44 | 7,7-Dimethyl-2-(4-trifluormethylphenyl)-6,7-dihydro-3H,5H-pyrrolo-[2,3-f]benzimidazol-6-on | >300 | -7 |
| 45 | 2'-Phenyl-spiro[cyclopentan-1,7'-6',7'-dihydro-3'H,5'H-pyrrolo-[2',3'-f]benzimidazol]-6'-on | 353-355 | -10 |

| Nr. | Verbindung | Schmp. [°C] | $\Delta$ E [$10^{-5}$M] |
|---|---|---|---|
| 46 | 2'-(2-Methoxy-4-hydroxy-phenyl)-spiro[cyclopentan-1,7'-6',7'-dihydro-3'H,5'H-pyrrolo[2',3'-f]-benzimidazol]-6'-on | 335-340 | -5 |
| 47 | 2'-(2-Methoxy-4-methylsulfonyl-phenyl)spiro[cyclopentan-1,7'-6',7'-dihydro-3'H,5'H-pyrrolo-[2',3'-f]benzimidazol]-6'-on | 270-272 | -5 |
| 48 | 7-Isopropyl-2-(4-methoxyphenyl)-6,7-dihydro-3H,5H-pyrrolo[2,3-f]-benzimidazol-6-on | 326-328 | -8 |
| 49 | 7,7-Dimethyl-2-phenylmethyl-6,7-dihydro-3H,5H-pyrrolo[2,3-f]benz-imidazol-6-on | 328-330 | -1 |
| 50 | 7,7-Dimethyl-2-(2-methoxyphenyl)-6,7-dihydro-3H,5H-pyrrolo[2,3-f]-benzimidazol-6-on | 300-303 | -10 |
| 51 | 7,7-Dimethyl-2-(4-methoxyphenyl)-6,7-dihydro-3H,5H-pyrrolo[2,3-f]-benzimidazol-6-on | 341-343 | -7 |
| 52 | 7,7-Dimethyl-2-(4-chlorphenyl)-6,7-dihydro-3H,5H-pyrrolo[2,3-f]-benzimidazol-6-on | 360-363 | -3 |
| 53 | 7,7-Dimethyl-2-(2-methoxy-5-methyl-sulfonyl-phenyl)-6,7-dihydro-3H,5H-pyrrolo[2,3-f]-benzimidazol-6-on | 236-238 | -2 |
| 54 | 7,7-Dimethyl-2-(4-methylphenyl)-6,7-dihydro-3H,5H-pyrrolo[2,3-f]-benzimidazol-6-on | >300 | -10 |
| 55 | 7,7-Diethyl-2-(4-methoxyphenyl)-6,7-dihydro-3H,5H-pyrrolo[2,3-f]-benzimidazol-6-on | 217-219 | -4 |
| 56 | 2'-(4-Methoxyphenyl)spiro[cyclopentan-1,7'-6',7'-dihydro-3'H,5'H-pyrrolo[2',3'-f]benzimidazol]-6'-on | 354-355 | -10 |

| Nr. | Verbindung | Schmp. [°C] | Δ E [$10^{-5}$M] |
|---|---|---|---|
| 57 | 7-Methyl-2-(2,4-dimethoxyphenyl)-6,7-dihydro-3H,5H-pyrrolo[2,3-f]-benzimidazol-6-on | 294-297 | -2 |
| 58 | 7,7-Dimethyl-2-(4-hydroxyphenyl)-6,7-dihydro-3H,5H-pyrrolo[2,3-f]-benzimidazol-6-on | 240 | -12 |
| 59 | 7,7-Dimethyl-2-phenyl-6,7-dihydro-3H,5H-pyrrolo[2,3-f]-benzimidazol-6-on | 195-220 | -8 |
| 60 | 7,7-Dimethyl-2-(2,4-dimethoxy-phenyl)-6,7-dihydro-3H,5H-pyrrolo-[2,3-f]benzimidazol-6-on | 294-297 | -10 |
| 61 | 7,7-Dimethyl-2-(3,4-dimethoxyphenyl)-6,7-dihydro-3H,5H-pyrrolo-[2,3-f]benzimidazol-6-on | 314-317 | -14 |
| 62 | 7,7-Dimethyl-2-(2-methoxy-4-chlor-phenyl)-6,7-dihydro-3H,5H-pyrrolo-[2,3-f]benzimidazol-6-on | 299-301 | -13 |
| 63 | 7,7-Dimethyl-2-[4-(1H-imidazol-1-yl)phenyl]-6,7-dihydro-3H,5H-pyrrolo[2,3-f]benzimidazol-6-on | 300 | -11 |
| 64 | 7,7-Dimethyl-2-(4-dimethylamino-phenyl-6,7-dihydro-3H,5H-pyrrolo-[2,3-f]benzimidazol-6-on | 262-270 | -8 |
| 65 | 7,7-Dimethyl-2-(2-methoxy-4-methyl-sulfonyloxy-phenyl)-6,7-dihydro-3H,5H-pyrrolo[2,3-f]benzimidazol-6-on | 233-235 | -8 |
| 66 | 7-Ethoxycarbonyl-7-methyl-2-phenyl-6,7-dihydro-3H,5H-pyrrolo[2,3-f]-benzimidazol-6-on | 178-182 | -3 |
| 67 | 7,7-Dimethyl-2-(2-methoxy-4-methyl-sulfonyl-phenyl)-6,7-dihydro-3H,5H-pyrrolo[2,3-f]benzimidazol-6-on | 235-237 | -10 |

| Nr. | Verbindung | Schmp. [°C] | ΔE [$10^{-5}$M] |
|---|---|---|---|
| 68 | 6-Phenyl-1-methyl-1,2,3,5-tetrahydrobenzo[1,2-d:4,5-d']diimidazol-2-on | >300 | -1 |
| 69 | 6-(4-Dimethylaminophenyl)-1-methyl-1,2,3,5-tetrahydrobenzo[1,2-d-4,5-d']diimidazol-2-on | 238-241 | -5 |
| 70 | 6-(4-(1-Imidazolyl)phenyl)-1-methyl-1,2,3,5-tetrahydrobenzo-[1,2-d:4,5-d']diimidazol-2-on | 260 Zers. | -5 |
| 71 | 6-(4-Diethylamino-2-methoxyphenyl)-1-methyl-1,2,3,5-tetrahydrobenzo-[1,2-d:4,5-d']diimidazol-2-on | >300 | -9 |
| 72 | 6-(4-Pyridyl)-1-ethyl-1,2,3,5-tetrahydrobenzo-[1,2-d:4,5-d']diimidazol-2-on | 237-239 | -3 |
| 73 | 6-(4-Pyridyl)-1-(1-propyl)-1,2,3,5-tetrahydrobenzo-[1,2-d:4,5-d']diimidazol-2-on | 235-237 | -5 |
| 74 | 6-(4-Pyridazinyl)-1-methyl-1,2,3,5-tetrahydrobenzo-[1,2-d:4,5-d']diimidazol-2-on | >300 | -2 |
| 75 | 6-(4-Pyridazinyl-1-ethyl-1,2,3,5-tetrahydrobenzo-[1,2-d:4,5-d']diimidazol-2-on | >300 | -4 |
| 76 | 6-(3-Thienyl)-2,3-dihydro-5H-imidazo[4,5-f]benzoxazol-2-on | 295-300 | -2 |
| 77 | 6-(4-Pyridyl)-2,3-dihydro-5H-imidazo[4,5-f]benzthiazol-2-on | >300 | |
| 78 | 6-Pyrazinyl-2,3-dihydro-5H-imidazo[4,5-f]benzoxazol-2-on | 335 | -2 |
| 79 | 6-(2-Methylmercaptophenyl)-2,3-dihydro-5H-imidazo[4,5-f]-benzoxazol-2-on | 208-211 | -2 |
| 80 | 6-(2-Methoxyphenyl)-2,3-dihydro-5H-imidazo[4,5-f]benzoxazol-2-on | 319-321 | -5 |

| Nr. | Verbindung | Schmp. [°C] | ΔE [$10^{-5}$M] |
|---|---|---|---|
| 81 | 6-Phenyl-2,3-dihydro-5H-imidazo-[4,5-f]benzoxazol-2-on | 298-300 | -4 |
| 82 | 6-(2-Methyl-4-pyridyl)-2,3-dihydro-5-H-imidazo[4,5-f]benzoxazol-2-on | >300 | -3 |
| 83 | 6-(1-Propyl)-2,3-dihydro-5H-imidazo[4,5-f]benzoxazol-2-on | 177-180 | -2 |
| 84 | 3,3-Dimethyl-6-(4-pyridyl)-benzo-[1,2-b:5,4-b']dipyrrol-2-(1H,3H,-7H)-on | >300 | -3 |
| 85 | 3,3-Dimethyl-6-(3-pyridyl)-benzo[1,2-b:5,4-b']dipyrrol-2-1H,3H,7H)-on x $CH_3OH$ | 305 | -6 |
| 86 | 3,3-Dimethyl-6-[4-(1H-imidazol-1-yl-phenyl]benzo[1,2-b:5,4-b']-dipyrrol-2-(1H,3H,7H)-on | >340 | -6 |
| 87 | 3-Methyl-6-(3-pyridyl)benzo-[1,2-b:5,4-b']dipyrrol-2-(1H,3H,-7H)-on | 330-331 | -2 |
| 88 | 3-Methyl-6-(3-pyridyl)benzo-[1,2-b:5,4-b']dipyrrol-2-(1H,3H,-7H)-on | 270-273 | -10 |
| 89 | 3,3-Dimethyl-6-(4-methylphenyl)-benzo[1,2-b:5,4-b']dipyrrol-2-1H,3H,7H)-on | 278-281 | -4 |
| 90 | 7,7-Dimethyl-2-cyclohexyl-6,7-dihydro-3H,5H-pyrrolo[2,3-f]-benz-imidazol-6-on | 315-318 | -4 |
| 91 | 2'-Propyl-spiro[cyclopentan-1,7'-6',7'-dihydro-3'H,5'H-pyrrolo-[2',3'-f]benzimidazol]-6'-on | 332-335 | -6 |
| 92 | 2-(1-Propenyl)-7,7-dimethyl-6,7-dihydro-3H,5H-pyrrolo[2,3-f]benz-imidazol-6-on | 252-254 | -7 |
| 93 | 2-n-Hexyl-7,7-dimethyl-6,7-dihydro-3H,5H-pyrrolo[2,3-f]benzimidazol-6-on | 233-234 | -8 |

| Nr. | Verbindung | Schmp. [°C] | ΔE [$10^{-5}$M] |
|---|---|---|---|
| 94 | 7,7-Dimethyl-2-(1-cyclopenten-1-yl)-6,7-dihydro-3H,5H-pyrrolo-[2,3-f]benzimidazol-6-on | 233-235 | -6 |
| 95 | 2-(4-Pyridylcarbonylamino)-7,7-dimethyl-6,7-dihydro-3H,5H-pyrrolo[2,3-f]benzimidazol-6-on | >300 | -7 |
| 96 | 2'-(4-Ethoxyphenyl)spiro [cyclo-pentan-1,7'-6',7'-dihydro-3'H,5'H-pyrrolo[2',3'-f]-benzimidazol]-6'-on, hergestellt nach EP 186 010 | 300 | -9 |

## Ansprüche

1. Verwendung von Verbindungen der allgemeinen Formel I

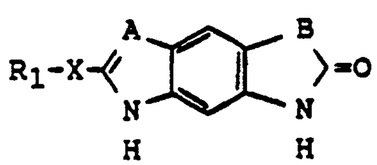

(I)

in welcher
$R_1$ einen Phenylring der allgemeinen Formel II darstellt,

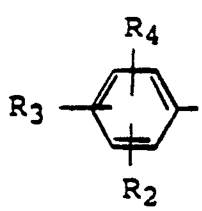

(II),

wobei $R_2$, $R_3$, $R_4$ gleich oder verschieden sein können und jeweils Wasserstoff, eine Alkansulfonyloxy-, Trifluormethansulfonyloxy-, Alkansulfonylamino-, Trifluormethansulfonylamino-, N-Alkyl-alkansulfonylamino-, N-Alkyltrifluormethansulfonylamino-, Alkylsulfenylmethyl-, Alkylsulfinylmethyl- oder Alkylsulfonylmethylgruppe, eine durch eine Hydroxy-, Alkoxy-, Amino-, Alkylamino- oder Dialkylaminogruppe substituierte Carbonylgruppe, eine durch eine Amino-, Alkylamino-, Dialkylamino- oder cyclische Iminogruppe substituierte Sulfonylgruppe, wobei eine Methylengruppe in 4-Stellung durch ein Schwefel- oder Sauerstoffatom ersetzt sein kann, eine Alkylcarbonylamino-, Aminocarbonylamino- oder Alkylaminocarbonylaminogruppe, eine Alkylmercapto-, Alkylsulfinyl- oder Alkylsulfonylgruppe, eine Nitro-, Halogen-, Amino-, Hydroxy-, Alkyl-, Alkoxy-, Alkenyloxy-, Alkinyloxy-, Cyanoalkyloxy-, Carboxyalkoxy-, Alkoxycarbonylalkyloxy-, Dialkylamino-, 1-Imidazolyl-, Trifluormethyl- oder Cyangruppe sein können,
oder einen heterocyclischen Fünfring mit 1-4 Heteroatomen oder einen heterocyclischen Sechsring mit 1-5 Heteroatomen darstellt, wobei die Heteroatome gleich oder verschieden sein können und Sauerstoff,

Schwefel oder Stickstoff bedeuten und gewünschtenfalls an einem oder mehreren Stickstoffatomen ein Sauerstoffatom tragen können, und die Fünf- oder Sechsringe gegebenenfalls durch eine oder mehrere Alkyl-, Alkoxy, Alkylmercapto-, Hydroxy-, Nitro-, Amino-, Halogen- oder Cyangruppen substituiert sein können,

oder für den Fall, daß X eine Bindung darstellt, $R_1$ neben den oben genannten Gruppen auch ein Wasserstoffatom, eine Alkyl-, Cycloalkyl-, Alkenyl-, Cycloalkenyl-, Alkinyl-, Halogenalkyl-, Alkoxyalkyl-, Carboxylalkyl-, Alkoxy-carbonylalkyl-, eine Hydroxy-, Mercapto-, Amino-, Alkylmercapto-, Formylaminoalkyl-, oder eine Pyridylcarbonyl-aminogruppe bedeutet,

X eine Bindung, eine Alkylen-, oder die Vinylengruppe bedeutet,

A ein Stickstoffatom oder die Gruppe $-\dot{C}R_5$ sein kann, wobei $R_5$ ein Wasserstoffatomen, eine Alkyl-, Alkenyl-, Cycloalkyl-, Cycloalkenyl, Cyan-, Alkylcarbonyl-, Alkoxycarbonyl-, Carboxy-, Aminocarbonyl-, Alkylaminocarbonyl-, Dialkylaminocarbonyl- oder Arylgruppe bedeutet,

B ein Sauerstoffatom, ein Schwefelatom oder die Gruppe $>NR_6$ bedeutet, wobei $R_6$ ein Wasserstoffatom oder eine Alkylgruppe bedeutet, oder B die Gruppe $>CR_7R_8$ sein kann, wobei $R_7$ ein Wasserstofatom, eine Alkyl-, Alkenyl-, oder eine Cycloalkylgruppe bedeutet, und $R_8$ ein Wasserstoffatom, eine Alkyl-, Alkenyl- oder Cyangruppe, eine durch eine Hydroxy-, Alkyl-, Alkoxy-, Amino-, Alkylamino-, Dialkylamino- oder Hydrazinogruppe substituierte Carbonylgruppe, oder $R_7$ und $R_8$ zusammen eine Alkyliden-oder Cycloalkylidengruppe bilden, oder $R_7$ und $R_8$ zusammen mit dem C-Atom, an das sie gebunden sind einen Spirocyclus bilden,

oder deren Tautomeren, optisch aktiven Formen und racemischen Gemischen, oder deren physiologisch verträglichen Salzen anorganischer und organischer Säuren, zur Herstellung von Arzneimitteln mit erythrozytenaggregations-hemmender Wirkung.

2. Verwendung von Verbindungen der allgemeinen Formel I gemäß Anspruch 1,

in welcher

$R_1$ den Phenylrest der allgemeinen Formel II bedeutet, in der

$R_2$ Wasserstoff, die Methansulfonyloxy-, Trifluormethansulfonyloxy-, Methansulfonylamino-, Trifluormethansulfonylamino-, Methansulfonyl-methylamino-, Trifluormethansulfonyl-methylamino-, Methylsulfinylmethyl-, Methylsulfonylmethyl-, Aminocarbonyl-, Aminosulfonyl-, Methylaminosulfonyl-, Dimethylaminosulfonyl-, Acetylamino-, Methylmercapto-, Methylsulfinyl-, Methylsulfonyl-, Hydroxy-, Allyloxy-, Methyl-, Methoxy-, Propargyloxy-, Cyanmethyl-, oxy-, Methoxycarbonyl-methyloxy-, Cyan-, Chlor-, Nitro-, Amino-, Dimethylamino-, Trifluormethyl- oder die 1-Imidazolylgruppe,

$R_3$ Wasserstoff, die Methyl-, Methoxy-, die Hydroxy-, Dimethylamino- oder Chlorgruppe bedeutet,

$R_4$ Wasserstoff oder die Methoxygruppe ist

oder

$R_1$ den Pyrrol-, Furan-, Thiophen-, Pyrazol-, Imidazol-, Isothiazol-, Thiazol-, Oxazol-, Triazol-, Tetrazol-, Thiadiazol-, Isoxazol-, Oxadiazol-, Pyridin-, N-Oxy-pyridin-, Pyrazin-, Pyrimidin-, N,N′-Dioxypyrimidin-, Pyridazin-, Oxazin-, Thiazin-, Triazin- oder Tetrazinrest darstellt, sowie deren Methyl-, Ethyl-, Methoxy-, Ethoxy-, Methylmercapto-, Ethylmercapto- und chlorsubstituierten Derivate,

oder

$R_1$ für den Fall, daß X eine Bindung darstellt, neben den genannten Gruppen auch ein Wasserstoffatom, die Methyl-, Ethyl-, Propyl-, Butyl-, Pentyl-, Hexyl-, Propenyl-, Cyclopentenyl-, Cyclohexyl-, Trifluormethyl-, Hydroxy-, Mercapto-, Methylmercapto-, Aminogruppe bedeutet,

X eine Bindung, die Ethylen- oder Vinylgruppe bedeutet,

A ein Stickstoffatom oder die - $\dot{C}$ H-Gruppe bedeutet,

B ein Sauerstoffatom, Schwefelatom, oder die Gruppe $>NR_6$ bedeutet, wobei $R_6$ die Methyl-, Ethyl- oder Propylgruppe bedeutet, oder B die Gruppe $>CR_7R_8$ bedeutet, wobei $R_7$ ein Wasserstoffatom, oder die Methylgruppe darstellt und $R_8$ die Methyl-, Ethyl- oder Isopropylgruppe bedeutet, oder $R_7$ und $R_8$ zusammen mit dem C-Atom, an das sie gebunden sind, einen Spirocyclopentylring darstellen.

3. Verwendung von Verbindungen der allgemeinen Formel I gemäß Anspruch 1, in der A in ein Stickstoffatom oder die Gruppe =CH- bedeutet.

4. Verwendung von Verbindungen der allgemeinen Formel I gemäß Anspruch 1, in der B die Iminogruppe -NH- oder die Gruppe $=CH_7R_8$ bedeutet, wobei $R_7$ ein Wasserstoffatom oder eine Alkylgruppe bedeutet und $R_8$ ein Wasserstoffatom, ein Alkyl-, Cyan-, Alkoxycarbonyl- oder Hydrazinocarbonylgruppe, oder $R_7$ und $R_8$ zusammen mit dem C-Atom, an das sie gebunden sind, einen $C_3$-$C_7$-Spirocycloalkylring bilden.

5. Verwendung von Verbindungen der allgemeinen Formel I gemäß Anspruch 1, in der X einen Valenzstrich bedeutet.

6. Verwendung von Verbindungen der allgemeinen Formel I gemäß Anspruch 1, in der $R_2$, $R_3$, $R_4$ ein Wasserstoffatom, eine Halogen-, Hydroxy-, Alkyl-, Alkoxy-, Alkenyloxy-oder Trifluormethylgruppe bedeutet.

7. Verwendung von
- 7,7-Dimethyl-2-(4-(2-Chlor-pyridyl))-6,7-dihydro-3H, 5H-pyrrolo-[2,3-f]benzimidazol-6-on
- 2′-(4-Pyridyl)-spiro[cyclopentan-1,7′-6,7′-dihydro-3′H,5′H-pyrrolo-[2′,3′-f]benzimidazol-6′-on
- 7,7-Dimethyl-2-(3,4-dichlorphenyl)-6,7-dihydro-3H,5H-pyrrolo-[2,3-f]-benzimidazol-6-on
- 7,7-Dimethyl-2-(2-phenyl-vinyl)-6,7-dihydro-3H,5H-pyrrolo-[2,3-f]-benzimidazol-6-on
- 7,7-Dimethyl-2-(4-trifluormethylphenyl)-6,7-dihydro-3H,5H-pyrrolo-[2,3-f]benzimidazol-6-on
- 2′-Phenyl-spiro[cyclopentan-1,7′-6′,7′-dihydro-3′H,5′H-pyrrolo-[2′,3′-f]benzimidazol]-6′-on
- 2′-(4-Methoxyphenyl)spiro[cyclopentan-1,7′-6′,7′-dihydro-3′H,5′H-pyrrolo-[2′,3′-f]benzimidazol]-6′-on
- 7,7-Dimethyl-2-(3,4-dimethoxyphenyl)-6,7-dihydro-3H,5H-pyrrolo-[2,3-f]benzimidazol-6-on

oder deren physiologisch verträglichen Salzen zur Herstellung von Arzneimitteln mit erythrozytenaggregations-hemmender Wirkung.

8. Verwendung von Verbindungen gemäß der Ansprüche 1-7 zur Herstellung von Arzneimitteln für die Behandlung von peripheren, coronaren oder cerebralen Durchblutungsstörungen, Schockzuständen, degenerativen Gefäßerkrankungen, rheumatischen Erkrankungen, Ulcera, nekrotischen Prozessen in Tumoren, degenerativen Störungen der Retina, Nerven oder Muskeln, oder von Hautkrankheiten, arteriellen Verschlußkrankheiten, Stenosen, ischämischen Zuständen, venöser Insuffizienz oder Diabetes mellitus.